# EUROPÄISCHE PATENTANMELDUNG

(11) **EP 0 867 168 A2**
(43) Veröffentlichungstag der Anmeldung: **30.09.1998**
(21) Anmeldenummer: 98105393.7
(22) Anmeldetag: 25.03.1998
(51) Int. Cl.: A61K 7/06, A61K 7/48

(54) **Topische Zubereitungen mit oxidationsempfindlichen Wirkstoffen**

(30) Priorität: 29.03.1997 DE 19713368
(71) Anmelder: Beiersdorf Aktiengesellschaft, 20245 Hamburg (DE)
(72) Erfinder: Schmidt-Lewerkühne, Hartmut, Dr., 22869 Schenefeld (DE); Riedel, Jan-Henric, Dr., 22307 Hamburg (DE)

(57) **Zusammenfassung**

Gegenstand der Erfindung sind kosmetische und dermatologische topische Zubereitungen mit einem oxidationsempfindlichen Wirkstoff oder mehreren oxidationsempfindlichen Wirkstoffen in verkapselter Form in Kombination mit einer Verbindung oder mehreren Verbindungen aus der Gruppe der Komplexbildner.

## Beschreibung

Die vorliegende Erfindung betrifft kosmetische und dermatologische topische Zubereitungen mit oxidationsempfindlichen Wirkstoffen. Insbesondere betrifft die vorliegende Erfindung solche Zubereitungen zur Pflege des Haares und der Kopfhaut. In einer bevorzugten Ausführungsform betrifft die vorliegende Erfindung Zubereitungen, die dazu dienen, das einzelne Haar zu pflegen, zu schützen und zu kräftigen und/oder der Haartracht insgesamt Halt und Fülle zu verleihen.

Das menschliche Haar kann, grob verallgemeinert, unterteilt werden in den lebenden Teil, die Haarwurzel, und den toten Teil, den Haarschaft. Der Haarschaft seinerseits besteht aus der Medulla, welche allerdings entwicklungsgeschichtlich bedingt für den neuzeitlichen Menschen unbedeutend geworden und zurückgebildet ist und bei dünnem Haar oft gänzlich fehlt, ferner dem die Medulla umschließenden Cortex und der die Gesamtheit aus Medulla und Cortex umhüllenden Cuticula.

Insbesondere die Cuticula als Außenhülle des Haares ist besonderer Beanspruchung durch Umwelteinflüsse, durch Kämmen und Bürsten, aber auch durch Haarbehandlung, insbesondere Haarfärbung und Haarverformung, z.B. Dauerwellverfahren, ausgesetzt.

Bei besonders aggressiver Beanspruchung, beispielsweise der Bleichung mit Oxidantien wie Wasserstoffperoxid, bei welcher die im Cortex verteilten Pigmente oxidativ zerstört werden, kann auch das Innere des Haars in Mitleidenschaft gezogen werden. Soll menschliches Haar dauerhaft gefärbt werden, kommen in der Praxis lediglich oxidierende Haarfärbeverfahren in Betracht. Beim oxidativen Haarfärben erfolgt die Ausbildung des Farbstoffchromophoren durch Reaktion von Präkursoren (Phenole, Aminophenole, seltener auch Diamine) und Basen (meistens p-Phenylendiamin) mit dem Oxidationsmittel, zumeist Wasserstoffperoxid. Wasserstoffperoxidkonzentrationen um 6% werden dabei gewöhnlich verwendet.

Üblicherweise wird davon ausgegangen, daß neben der Färbewirkung auch eine Bleichwirkung durch das Wasserstoffperoxid erfolgt. In oxidativ gefärbtem menschlichem Haar sind, ähnlich wie bei gebleichtem Haar, mikroskopische Löcher an den Stellen, an denen Melaningranula vorlagen, nachweisbar. Tatsache ist, daß das Oxidationsmittel Wasserstoffperoxid nicht nur mit den Farbvorstufen, sondern auch mit der Haarsubstanz reagieren und dabei unter Umständen eine Schädigung des Haares bewirken kann.

Auch die Haarwäsche mit aggressiven Tensiden kann das Haar beanspruchen, zumindest dessen Erscheinungsbild oder das Erscheinungsbild der Haartracht insgesamt herabsetzen. Beispielsweise können bestimmte wasserlösliche Haarbestandteile durch die Haarwäsche herausgelaugt werden.

Aus diesen Gründen werden seit geraumer Zeit teils Haarpflegekosmetika verwendet, welche dazu bestimmt sind, nach Einwirken aus dem Haar wieder ausgespült zu werden, teils solche, welche auf dem Haar verbleiben sollen. Letztere können so formuliert werden, daß sie nicht nur der Pflege des einzelnen Haars dienen, sondern auch das Aussehen der Haartracht insgesamt verbessern, beispielsweise dadurch, daß sie dem Haar mehr Fülle verleihen, die Haartracht über einen längeren Zeitraum fixieren oder seine Frisierbarkeit verbessern.

Durch quaternäre Ammoniumverbindungen beispielsweise läßt sich die Kämmbarkeit der Haare entscheidend verbessern. Solche Verbindungen ziehen auf das Haar auf und sind oft noch nach mehreren Haarwäschen auf dem Haar nachweisbar.

Der Stand der Technik ließ es aber an Wirkstoffen und Zubereitungen mangeln, welche dem geschädigten Haar in befriedigender Weise Pflege zukommen ließen. Auch erwiesen sich Zubereitungen, die der Haartracht Fülle geben sollten, oft als unzureichend, zumindest waren sie ungeeignet, als Haarpflegezubereitungen eingesetzt zu werden. Die Haartracht fixierende Zubereitungen des Standes der Technik enthalten beispielsweise in der Regel viskose Bestandteile, welche Gefahr laufen, ein Gefühl der Klebrigkeit zu erwecken, welches oft durch geschickte Formulierung kompensiert werden muß.

Auch die Haut, insbesondere die Altershaut, aber auch die Kopfhaut ist chemischen und physikalischen Streßfaktoren (Toxinen, UV-Licht) ausgesetzt.

Zur Behandlung der Schäden und auch vorbeugend ist es bekannt, den genannten Zubereitungen als Wirkstoffe Antioxidantien z.B. Vitamine zuzugeben. Dabei ist es ein großes Problem, Grundlage und Wirkstoffe so abzustimmen, daß die Wirkstoffe eine ausreichende Lagerstabilität erlangen, denn diese Wirkstoffe besitzen eine unterschiedliche Empfindlichkeit gegenüber oxidativen Einflüssen und Oxidationsmittel (Sauerstoff, Licht) und Wärme.

Als Lösungen dieses Problems ist schon vorgeschlagen worden, die Wirkstoffe durch die Zugabe eines oder mehrerer Stabilisatoren, z.B. anderen Antioxidantien vor schnellem Abbau zu bewahren oder sie in der Form stabiler Derivate einzusetzen. Häufig bleibt aber die erzielte Wirkung hinter der erhofften Wirkung zurück.

Aufgabe der Erfindung war es daher, hier Abhilfe zu schaffen und stabile topische Zubereitungen mit oxidationsempfindlichen Wirkstoffen zu schaffen, deren Wirkstoffgehalt über einen langen Zeitraum erhalten bleibt.

Die Aufgaben werden erfindungsgemäß gelöst.

Gegenstand der Erfindung sind kosmetische und dermatologische topische Zubereitungen mit einem oxidationsempfindlichen Wirkstoff oder mehreren oxidationsempfindlichen Wirkstoffen in verkapselter Form in Kombination mit einer Verbindung oder mehreren Verbindungen aus der Gruppe der Komplexbildner.

Gegenstand der Erfindung ist auch die Verwendung von einem oxidationsempfindlichen Wirkstoff oder mehreren oxidationsempfindlichen Wirkstoffen in verkapselter Form in Kombination mit einer Verbindung oder mehreren Verbindungen aus der Gruppe der Komplexbildner in kosmetischen und dermatologischen Zubereitungen.

Nachdem die Zubereitungen mit den Komplexbildnern auf die technisch übliche Art und Weise hergestellt und homogenisiert worden sind, werden im letzten Herstellungsschritt die Kapseln mit dem zu schützenden Wirkstoff dazugegeben und vorsichtig homogen untergerührt. Danach können die fertigen Zubereitungen gelagert werden. Zu beachten ist hierbei, daß die Verkapselungsmaterialien durch die einsetzende Hydratisierung stark erweichen (zwischen 1 h und 1 Tag nach Herstellung), was beim Auftreten von stärkeren Scherkräften während des Abfüllprozesses zu einer Zerstörung der Strukturen führen kann. Vorzugsweise verwendet man zur schonenden Abfüllung daher Kapselpumpen. Der Reifungsprozeß ist zweckmäßig, denn dadurch werden die Kapseln auf der Haut und dem Humanhaar gut verteilbar und somit kann der Wirkstoff homogen verteilt werden.

Es ist auch möglich, die fertigen Zubereitungen vor dem Beginn des Hydratisierungsprozesses abzufüllen. Die Wasseraufnahme kann dann anschließend in der Endverpackung erfolgen.

Gegenstand der Erfindung ist auch das Verfahren zur Herstellung der erfindungsgemäßen topischen Zubereitungen, das dadurch gekennzeichnet ist, daß man in an sich bekannter Weise ohne Wirkstoffe die kosmetischen oder dermatologischen topischen Zusammensetzungen herstellt, und dann erst zum Schluß die Wirkstoffkapseln zufügt und in einem schonenden Mischverfahren mit der Grundlage vermischt.

Bevorzugt werden wasserhaltige topische Zubereitungen, z.B. Emulsionen oder Gele verwendet. Den an sich weitgehend wasserfreien Zubereitungsformen wie z.B. Ölen oder Fettsalben können geringe Mengen Wasser zugegeben werden, wobei die Menge von der Menge des Kapselmaterials abhängt, und gegebenenfalls vom Fachmann in einem kurzen Vorversuch ermittelt wird. Beispielsweise reichen Wassermengen von 0,1 bis zu 5 Gew.-%, insbesondere 1 bis 3 Gew.-%, jeweils bezogen auf das Gesamtgewicht der Zubereitungen, für eine ausreichende Hydratisierung der Kapselhüllen.

Gegenstand der Erfindung ist auch die Verwendung von Komplexbildnern zur Stabilisierung von verkapselten oxidationsempfindlichen Wirkstoffen in kosmetischen und dermatologischen Zubereitungen.

Die erfindungsgemäßen Zubereitungen sind für die Behandlung der Haut sowie der Kopfhaut und insbesondere der Haare geeignet und werden in der für topische kosmetische und dermatologische Mittel üblichen Weise angewendet.

Oxidationsempfindliche Wirkstoffe sind solche Wirkstoffe, die nur in stabilisierter Form in den Zubereitungen eine ausreichende Lagerstabilität erlangen, z. B. wenn sie vor Oxidation durch den Luftsauerstoff geschützt sind. Wichtige Wirkstoffe dieser Art sind daher z.B. die Antioxidantien und darunter insbesondere Vitamine.

Erfindungsgemäße bevorzugte Antioxidantien sind insbesondere Vitamin A und seine Derivate, die allgemein als Retinoide bezeichnet werden und Vitamin E und seine Derivate, insbesondere solche, die zu der Substanz-Klasse der Tocopherole zählen.

Die Retinoide und Tocopherole sind in der Literatur beschrieben (Römpp Chemie Lexikon, 9. Auflage, Georg Thieme Verlag, Stuttgart, Seiten 3855 ff und 4637 ff).

Geeignete Derivate der erfindungsgemäßen Verbindungen sind insbesondere die Salze und Säureadditionssalze. Ester von Carbonsäuregruppen der Wirkstoffe mit Alkoholen oder Ester der OH-Gruppen mit Carbonsäuren sind ebenfalls geeignete Derivate.

Bevorzugte Salze sind wasserlösliche Salze, z.B. Natrium, Kalium- und Ammoniumsalze. Dies gilt auch für die Säureadditionssalze. Geeignete Säureadditionssalze werden z.B. mit anorganischen und organischen Säuren erhalten. Bevorzugt werden die Hydrochloride, Phosphate, Sulfate, Acetate, Caprylate, Zitrate, Lactate, Malate oder Tartrate.

Geeignete Ester sind z.B. solche, die mit kurzkettigen, mittelkettigen oder langkettigen Carbonsäuren oder Alkoholen gebildet werden, vorzugsweise mit mono-Alkoholen oder mono-Carbonsäuren. Beide Reste können geradkettig oder verzweigt sein und vorzugsweise 1 bis 20 Kohlenstoffatome besitzen.

Besonders bevorzugte Retinoide sind:
Vitamin A (-Alkohol, Retinol, z.B. 11-cis- oder 13-cis-Retinol)
Vitamin-A-Aldehyd (Retinal)
Vitamin-A-Säure (Tretinoin, Iso-Tretinoin)
Motretinid
Etretinat
Retinol-Ester, z.B. Retinyl-acetat, insbesondere Retinyl-Palmitat.

Besonders bevorzugte Tocopherole sind Vitamin E und seine Ester, insbesondere Vitamin E-Acetat.

Die erfindungsgemäßen Wirkstoffe können z.B. in den Zubereitungen, bezogen auf den nicht verkapselten Wirkstoff, in Mengen von 0,00001 bis 50 Gew.-%, vorzugsweise 0,0001 bis 1 Gew.-%, insbesondere 0,001 bis 0,01 Gew-%, jeweils bezogen auf das Gesamt-Gewicht der Zubereitungen, enthalten sein. Entsprechend dem jeweiligen Wirkstoffgehalt der der Wirkstoff enthaltenden Kapseln, werden dann die Kapseln in solchen größeren Gewichtsmengen eingesetzt, daß die gewünschte Wirkstoffmenge erhalten wird.

Die verkapselten Wirkstoffe liegen als Wirkstoffdepots in Form von insbesondere makroskopischen Kapseln vor. Sie sind in dieser Form schon gegen den Einfluß von Oxidationsmitteln, beispielsweise den Luftsauerstoff, besser geschützt als der unverkapselte Wirkstoff. Dieser Oxidationsschutz allein reicht aber nicht aus, um die gewünschte Lagerstabilität in allen Zubereitungsformen zu gewährleisten.

Erfindugnsgemäße verkapselte Wirkstoffe sind bekannt, können nach bekannten Verfahren erhalten werden und sind im Handel erhältlich.

Geeignete Verkapselungsmaterialien sind Polysaccharide wie Dextrine, insbesondere Cyclodextrine, Cellulose, insbesondere Carboxymethylcellulose oder Methylcellulose, polymere Matrices aus Polyethylen, Polystyrol, Polypropylen, Polyamid, Poly(meth)acrylsäure und ihre Derivate, insbesondere ihre Ester mit kurzkettigen und mittelkettigen Alkoholen und ihre Salze.

Verkapselungsmaterialien auf Cellulosebasis z.B. Carboxymethylcellulose enthalten insbesondere Zuckeranteile wie Lactose oder Glucose in Anteilen von vorzugsweise bis zu 20 Gew.-%. Sie werden besonders bevorzugt.

In den Kapseln können die Wirkstoffe z.B. in Mengen von 0,01 bis 50 Gew.-%, vorzugsweise 0,1 bis 25 Gew.-%, insbesondere 1 bis 10 Gew.-%, jeweils bezogen auf das Gesamtgewicht der wirkstoffhaltigen Kapsel, enthalten sein.

Der Durchmesser der Kapseln liegt üblicherweise z.B. im Bereich von 0,1 - 3, insbesondere 0,5 - 2 mm.

Besonders bevorzugt werden die folgenden Verkapselungen:
1. Cyclosystem Complex (beta-Cyclodextrin), Instituto Ricerche Applicate (IRA) mit 25% Vitamin A-palmitat
2. Polyamide millicapsules, Fa. Lipotec mit 1,5% Vitamin E-acetat
3. Polyamide millicapsules, Fa. Lipotec mit 1,5% Vitamin E
4. Polyamide millicapsules, Fa. Lipotec mit 1,5% Vitamin A
5. Unisphären (Lactose, Cellulose, Hydroxypropylmethylcellulose), Fa. Induchem mit 1,5% Vitamin E-acetat
6. Unisphären (Lactose, Cellulose, Hydroxypropylmethylcellulose), Fa. Induchem mit 1,5% Vitamin E
7. Unisphären (Lactose, Cellulose, Hydroxypropylmethylcellulose), Fa. Induchem mit 1,5% Vitamin A
8. Phytosphären (Locust Bean Gum, Xanthan), Fa. Coletica
9. Phytosphären (Locust Bean Gum, Xanthan), Fa. Coletica mit 1,5% Vitamin A
10. Methyl-beta-Cyclodextrine mit Vitamin E-nicotinat

Die %-Angaben sind Gew.-%. Die Verkapselungsmaterialien sind jeweils angegeben.

Durch die erfindungsgemäße Kombination mit einem Komplexbildner werden die verkapselten Wirkstoffe in hohem Maße stabilisiert und gegen Oxidation geschützt.

Bevorzugt werden wasserlösliche Komplexbildner, insbesondere mit hohem Bindungsvermögen für Kupfer- und Eisenionen.

Besonders bevorzugt werden Komplexbildner auf der Basis von Aminopolycarbonsäuren und deren Salzen. Bevorzugte Salze sind die vorstehend schon erwähnten Salze und Säureadditionssalze.

Besonders bevorzugt werden die folgenden Komplexbildner und deren wasserlösliche Salze, z.B. die Natriumsalze: Ethylendiaminotetraessigsäure (EDTA), Nitrilotriessigsäure (NTA), Hydroxyethylethylendiaminotriessigsäure (HOEDTA), Diethylentriaminopentaessigsäure (DTPA), Trans-1,2-diaminocyclohexantetraessigsäure (CDTA).

Als Stabilisatoren oder weitere Hilfsstoffe können gegebenenfalls Zitronensäure, Weinsäure oder Salizylsäure zugegeben werden.

Die Komplexbildner können in den Zubereitungen z.B. in Mengen von 0,001 bis 20 Gew.-%, vorzugsweise 0,05 bis 5 Gew.-%, insbesondere 0,2 bis 2 Gew.-%, jeweils bezogen auf das Gesamtgewicht der Zubereitungen, enthalten sein.

Die erfindungsgemäßen Kombinationen und Zubereitungen, diese Wirkstoffe enthalten, pflegen beispielsweise durch Umwelteinflüsse geschädigtes oder strapaziertes Haar bzw. beugen solchen Umwelteinflüssen vor. Zudem verbessern die erfindungsgemäßen Wirkstoffkombinationen und Zubereitungen, diese Wirkstoffkombinationen enthaltend, die Kämmbarkeit des Haares, ohne daß ein Zusatz an Wirkstoffen, enthaltend quaternäre Stickstoffgruppen ( 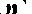 Quats") nötig wäre.

Außerdem dienen sie zum Schutz und zur Pflege der Haut.

Zur Anwendung werden die erfindungsgemäßen kosmetischen und dermatologischen Zubereitungen in der für Kosmetika üblichen Weise auf die Haut und/oder die Haare in ausreichender Menge aufgebracht. Bei der Anwendung der Zubereitungen werden die Wirkstoffkapseln mechanisch zerstört und geben den enthaltenen Wirkstoff frei.

Der pH-Wert der für die Haut und für die Haare bestimmten Zubereitungen kann z.B. in dem üblichen Bereich von 3 bis 9 liegen. Für Zubereitungen, die an der Haut angewendet werden wird der Bereich von pH 4 - 6 bevorzugt.

Vorteilhaft enthalten erfindungsgemäße Zubereitungen neben einem wirksamen Gehalt an erfindungsgemäßen Wirkstoffkombinationen ferner übliche Wirk-, Inhalts-, Zusatz- und/oder Hilfsstoffe.

Die erfindungsgemäßen kosmetischen und dermatologischen Zubereitungen können kosmetische Hilfsstoffe enthalten, wie sie üblicherweise in solchen Zubereitungen verwendet werden, z.B. Konservierungsmittel, Bakterizide, Parfüme, Substanzen zum Verhindern des Schäumens, Farbstoffe, Pigmente, die eine färbende Wirkung haben, Verdickungsmittel, oberflächenaktive Substanzen, Emulgatoren, weichmachende, anfeuchtende und/oder feuchhaltende Substanzen, Fette, Öle, Wachse oder andere übliche Bestandteile einer kosmetischen oder dermatologischen Formulierung wie Alkohole, Polyole, Polymere, Schaumstabilisatoren, Elektrolyte, organische Lösungsmittel oder Silikonderivate.

Weiterhin können in den Zubereitungen gegebenenfalls auch übliche Antioxidantien enthalten sein.

Die Menge der Antioxidantien (eine oder mehrere Verbindungen) in den Zubereitungen beträgt vorzugsweise 0,001 bis 30 Gew.-%, besonders bevorzugt 0,05 - 20 Gew.-%, insbesondere 1 - 10 Gew.-%, bezogen auf das Gesamtgewicht der Zubereitung.

Die wäßrige erfindungsgemäßen Zubereitungen enthalten gegebenenfalls vorteilhaft Alkohole, Diole oder Polyole niedriger C-Zahl, sowie deren Ether, vorzugsweise Ethanol, Isopropanol, Propylenglykol, Glycerin, Ethylenglykol, Ethylenglykolmonoethyl- oder -monobutylether, Propylenglykolmonomethyl, -monoethyl- oder -monobutylether, Diethylenglykolmonomethyl- oder -monoethylether und analoge Produkte, ferner Alkohole niedriger C-Zahl, z.B. Ethanol, Isopropanol, 1,2-Propandiol, Glycerin sowie insbesondere ein oder mehrere Verdickungsmittel, welches oder welche vorteilhaft gewählt werden können aus der Gruppe Siliciumdioxid, Aluminiumsilikate.

Gele gemäß der Erfindung enthalten z.B. üblicherweise Alkohole niedriger C-Zahl, z.B. Ethanol, Isopropanol, 1,2-Propandiol, Glycerin und Wasser bzw. ein vorstehend genanntes Öl in Gegenwart eines Verdickungsmittels, das bei ölig-alkoholischen Gelen vorzugsweise Siliciumdioxid oder ein Aluminiumsilikat, bei wäßrig-alkoholischen oder alkoholischen Gelen vorzugsweise ein Polyacrylat ist.

Vorteilhaft können erfindungsgemäße Zubereitungen außerdem Substanzen enthalten, die UV-Strahlung im UVB-Bereich absorbieren, wobei die Gesamtmenge der Filtersubstanzen z.B. 0.01 Gew.-% bis 30 Gew.-%, vorzugsweise 0,5 bis 10 Gew.-%, insbesondere 1,0 bis 6,0 Gew.-% beträgt, bezogen auf das Gesamtgewicht der Zubereitungen, um kosmetische Zubereitungen zur Verfügung zu stellen, die das Haar bzw. die Haut vor dem gesamten Bereich der ultravioletten Strahlung schützen. Sie können auch als Sonnenschutzmittel fürs Haar oder die Haut, insbesondere die Kopfhaut dienen.

Enthalten die erfindungsgemäßen Emulsionen UVB-Filtersubstanzen, können diese vorteilhaft wasserlöslich sein. Vorteilhafte wasserlösliche UVB-Filter sind z.B.:
- Salze der 2-Phenylbenzimidazol-5-sulfonsäure wie ihr Natrium-, Kalium- oder ihr Triethanolammonium-Salz, sowie die 2-Phenylbenzimidazol-5-sulfonsäure selbst;
- Sulfonsäure-Derivate von Benzophenonen, vorzugsweise 2-Hydroxy-4-methoxybenzophenon-5-sulfonsäure und ihre Salze;
- Sulfonsäure-Derivate des 3-Benzylidencamphers, wie z.B. 4-(2-Oxo-3-bornylidenmethyl)benzolsulfonsäure, 2-Methyl-5-(2-oxo-3-bornylidenmethyl)sulfonsäure und ihre Salze.

Es kann auch von Vorteil sein,erfindungsgemäße Zubereitungen mit UVA-Filtern zu versetzen, die bisher üblicherweise in kosmetischen Zubereitungen enthalten sind. Es können die für die UVB-Kombination verwendeten Mengen eingesetzt werden.

Bei kosmetischen und dermatologischen Zubereitungen gemäß der Erfindung handelt es sich beispielsweise um Shampoonierungsmittel, Zubereitungen, die beim Spülen der Haare vor oder nach der Shampoonierung, vor oder nach der Dauerwellbehandlung, vor oder nach der Färbung oder Entfärbung der Haare angewendet werden, um Zubereitungen zum Fönen oder Einlegen der Haare, Zubereitungen zum Färben oder Entfärben, um eine Frisier- und Behandlungslotion, einen Haarlack oder um Dauerwellmittel.

Die kosmetischen und dermatologischen enthalten Wirkstoffe und Hilfsstoffe, wie sie üblicherweise für diesen Typ von Zubereitungen zur Haarpflege und Haarbehandlung verwendet werden. Als Hilfsstoffe dienen Konservierungsmittel, oberflächenaktive Substanzen, Substanzen zum Verhindern des Schäumens, Verdickungsmittel, Emulgatoren, Fette, Öle, Wachse, organische Lösungsmittel, Bakterizide, Parfüme, Farbstoffe oder Pigmente, deren Aufgabe es ist, die Haare oder die kosmetische oder dermatologische Zubereitung selbst zu färben, Elektroyte, Substanzen gegen das Fetten der Haare.

Unter Elektrolyten im Sinne der vorliegenden Erfindung sind wasserlösliche Alkali-, Ammonium-Salze anorganischer Anionen und beliebige Gemische aus solchen Salzen zu verstehen, wobei gewährleistet sein muß, daß sich diese Salze durch pharmazeutische oder kosmetische Unbedenklichkeit auszeichnen.

Die erfindungsgemäßen Anionen werden bevorzugt gewählt aus der Gruppe der Chloride, der Sulfate und Hydrogensulfate, der Phosphate, Hydrogenphosphate und der linearen und cyclischen Oligophosphate sowie der Carbonate und Hydrogencarbonate.

Kosmetische Zubereitungen, die ein Shampoonierungsmittel darstellen, enthalten vorzugsweise mindestens eine anionische, nicht-ionische oder amphotere oberflächenaktive Substanz, oder auch Gemische aus solchen Substanzen, Chlorogensäure im wäßrigen Medium und Hilfsmittel, wie sie üblicherweise dafür verwendet werden. Die oberflächenaktive Substanz bzw. die Gemische aus diesen Substanzen können in einer Konzentration zwischen 1 Gew.-% und 50 Gew.-% in dem Shampoonierungsmittel vorliegen.

Liegen die kosmetischen oder dermatologischen Zubereitungen in Form einer Lotion vor, die ausgespült und z.B. vor oder nach der Entfärbung, vor oder nach der Shampoonierung, zwischen zwei Shampoonierungsschritten, vor oder nach der Dauerwellbehandlung angewendet wird, so handelt es sich dabei z.B. um wäßrige oder wäßrig-alkoholische Lösungen, die gegebenenfalls oberflächenaktive Substanzen enthalten, deren Konzentration zwischen 0,1 und 10 Gew.-%, vorzugsweise zwischen 0,2 und 5 Gew.-%, liegen kann.

Eine kosmetische Zubereitung in Form einer Lotion, die nicht ausgespült wird, insbesondere eine Lotion zum Einlegen der Haare, eine Lotion, die beim Fönen der Haare verwendet wird, eine Frisier- und Behandlungslotion, stellt im allgemeinen eine wäßrige, alkoholische oder wäßrig-alkoholische Lösung dar und enthält die erfindungsgemäßen Kombinationen..

Erfindungsgemäß können kosmetische Zubereitungen zur Behandlung und Pflege der Haare als Gele vorliegen, die organische Verdickungsmittel, z.B. Gummiarabikum, Xanthangummi, Natriumalginat, Cellulose-Derivate, vorzugsweise Methylcellulose, Hydroxymethylcellulose, Hydroxyethylcellulose, Hydroxypropylcellulose, Hydroxypropylmethylcellulose oder anorganische Verdickungsmittel, z.B. Aluminiumsilikate wie beispielsweise Bentonite, oder ein Gemisch aus Polyethylenglykol und Polyethylenglycolstearat oder -distearat, enthalten. Das Verdickungsmittel ist in dem Gel z.B. in einer Menge zwischen 0,1 und 30 Gew.-%, bevorzugt zwischen 0,5 und 15 Gew.-%, enthalten.

Erfindungsgemäße wäßrige kosmetische Reinigungsmittel oder für die wäßrige Reinigung bestimmte wasserarme oder wasserfreie Reinigungsmittelkonzentrate können anionische, nichtionische und/oder amphotere Tenside enthalten, beispielsweise herkömmliche Seifen, z.B. Fettsäuresalze des Natriums, Alkylsulfate, Alkylethersulfate, Alkan- und Alkylbenzolsulfonate, Sulfoacetate, Sulfobetaine, Sarcosinate, Amidosulfobetaine, Sulfosuccinate, Sulfobernsteinsäurehalbester, Alkylethercarboxylate, Eiweiß-Fettsäure-Kondensate, Alkylbetaine und Amidobetaine, Fettsäurealkanolamide, Polyglycolether-Derivate.

Kosmetische Zubereitungen, die kosmetische Reinigungszubereitungen für das Haar bzw. die Kopfhaut darstellen, können in flüssiger oder fester Form vorliegen. Sie enthalten vorzugsweise mindestens eine anionische, nicht-ionische oder amphotere oberflächenaktive Substanz oder Gemische daraus, gegebenenfalls einen Elektrolyten und Hilfsmittel, wie sie üblicherweise dafür verwendet werden. Die oberflächenaktive Substanz kann in einer Konzentration zwischen 1 und 94 Gew.-% in den Reinigungszubereitungen vorliegen, bezogen auf das Gesamtgewicht der Zubereitungen.

Die erfindungsgemäßen kosmetischen oder dermatologischen topischen Zubereitungen für die Haut können auf an sich üblichen Formulierungsgrundlagen beruhen und zur Behandlung der Haut im Sinne einer dermatologischen Behandlung oder einer Behandlung im Sinne der Kosmetik dienen.

Zur Anwendung werden die Zubereitungen in der für Kosmetika oder Dermatika üblichen Weise auf die Haut oder die Haare in ausreichender Menge einmal oder mehrmals täglich aufgebracht.

Besonders bevorzugt sind Hautpflegepräparationen und Sonnenschutz- Präparate.

Dermatologische und kosmetische Zubereitungen gemäß der Erfindung können in verschiedener Form vorliegen. So können z.B. wäßrige, alkoholische oder wäßrig-alkoholische Lösungen, Emulsionen vom Typ Öl-in-Wasser (O/W), Emulsionen vom Typ Wasser-in-Öl (W/O), multiple Emulsionen z.B. vom Typ Wasser-in Öl-in-Wasser (W/O/W), Gele, Hydrodispersionen, feste Stifte oder Aerosole die o.g. Wirkstoffkombinationen enthalten. Bevorzugt werden auch wasserarme oder wasserfreie Salben und Zubereitungen.

Vorteilhaft sind aber auch solche Zubereitungen, welche nach der Lichtexposition auf die Haut aufgetragen werden, also Après-Soleil-Produkte. Es liegt bei solchen Zubereitungen im Ermessen des Fachmannes, ob zusätzliche UV-Filtersubstanzen verwendet werden sollen oder nicht.

Kosmetische Zubereitungen gemäß der Erfindung zum Schutze der Haut vor UV-Strahlen können in verschiedenen Formen vorliegen, wie sie z.B. üblicherweise für diesen Typ von Zubereitungen eingesetzt werden. So können sie z.B. eine wäßrige, alkoholische oder wäßrig alkoholische Lösung, eine Emulsion vom Typ Wasser-in-Öl (W/O) oder vom Typ Öl-in-Wasser (O/W), oder eine multiple Emulsionen, beispielsweise vom Typ Wasser-in-Öl-in-Wasser (W/O/W), ein Gel, eine Hydrodispersion, ein Öl, einen festen Stift darstellen.

Die erfindungsgemäßen topischen Zubereitungen können dermatologische und kosmetische Hilfsstoffe enthalten, wie sie üblicherweise in solchen Zubereitungen verwendet werden, z.B. Konservierungsmittel, Bakterizide, Parfüme, Mittel zum Verhindern des Schäumens, Farbstoffe, Pigmente, die eine färbende Wirkung haben, Verdickungsmittel, oberflächenaktive Substanzen, Emulgatoren, weichmachende Substanzen, anfeuchtende und/oder feuchhaltende Substanzen, Fette, Öle, Wachse oder andere übliche Bestandteile einer kosmetischen Formulierung wie Alkohole, Polyole, Polymere, Schaumstabilisatoren, Elektrolyte, organische Lösungsmittel oder Silikonderivate.

Sofern die kosmetische oder dermatologische Zubereitung eine Lösung oder Lotion darstellt, können als Lösungsmittel verwendet werden:
- Wasser oder wäßrige Lösungen;
- Öle, wie Triglyceride der Caprin- oder der Caprylsäure, vorzugsweise aber Rizinusöl;
- Fette, Wachse und andere natürliche und synthetische Fettkörper, vorzugsweise Ester von Fettsäuren mit Alkoholen niedriger C-Zahl, z.B. mit Isopropanol, Propylenglykol oder Glycerin, oder Ester von Fettalkoholen mit Alkansäuren niedriger C-Zahl oder mit Fettsäuren;
- Alkohole, Diole oder Polyole niedriger C-Zahl, sowie deren Ether, vorzugsweise Ethanol, Isopropanol, Propylenglykol, Glycerin, Ethylenglykol, Ethylenglykolmonoethyl- oder -monobutylether, Propylenglykolmonomethyl, -monoethyl- oder -monobutylether, Diethylenglykolmonomethyl- oder -monoethylether und analoge Produkte.

Insbesondere werden Gemische der vorstehend genannten Lösungsmittel verwendet. Bei alkoholischen Lösungsmitteln kann Wasser ein weiterer Bestandteil sein.

Öle oder Emulsionen gemäß der Erfindung z.B. in Form einer Sonnenschutzcreme, einer Sonnenschutzlotion oder einer Sonnenschutzmilch sind vorteilhaft und enthalten z.B. die genannten Fette, Öle, Wachse und anderen Fettkörper, sowie Wasser und einen Emulgator, wie er üblicherweise für einen solchen Typ der Formulierung verwendet wird.

Kosmetische und dermatologische Zubereitungen zur Behandlung und Pflege der Haut können als Gele vorliegen, die neben den Wirkstoffen und dafür üblicherweise verwendeten Lösungsmitteln noch organische Verdickungsmittel, z.B. Gummiarabikum, Xanthangummi, Natriumalginat, Cellulose-Derivate, vorzugsweise Methylcellulose, Hydroxymethylcellulose, Hydroxyethylcellulose, Hydroxypropylcellulose, Hydroxypropylmethylcellulose oder anorganische Verdickungsmittel, z. B. Aluminiumsilikate wie beispielsweise Bentonite, oder ein Gemisch aus Polyethylenglykol und Polyethylenglykolstearat oder -distearat, enthalten. Das Verdickungsmittel ist in dem Gel z.B. in einer Menge zwischen 0,1 und 30 Gew.-%, bevorzugt zwischen 0,5 und 15 Gew.-%, enthalten.

Erfindungsgemäße Gele enthalten üblicherweise Alkohole niedriger C-Zahl, z.B. Ethanol, Isopropanol, 1,2-Propandiol, Glycerin und Wasser bzw. ein vorstehend genanntes Öl in Gegenwart eines Verdickungsmittels, das bei ölig-alkoholischen Gelen vorzugsweise Siliciumdioxid oder ein Aluminiumsilikat, bei wäßrig-alkoholischen oder alkoholischen Gelen vorzugsweise ein Polyacrylat ist.

Hydrodispersionen stellen Dispersionen einer flüssigen, halbfesten oder festen inneren (diskontinuierlichen) Lipidphase in einer äußeren wäßrigen (kontinuierlichen) Phase dar.

Im Gegensatze zu O/W-Emulsionen, die sich durch eine ähnliche Phasenanordnung auszeichnen, sind Hydrodispersionen aber im wesentlichen frei von Emulgatoren. Hydrodispersionen stellen, wie im übrigen auch Emulsionen, metastabile Systeme dar und sind geneigt, in einen Zustand zweier in sich zusammenhängender diskreter Phasen überzugehen. In Emulsionen verhindert die Wahl eines geeigneten Emulgators die Phasentrennung.

Bei Hydrodispersionen einer flüssigen Lipidphase in einer äußeren wäßrigen Phase kann die die Stabilität eines solchen Systems beispielsweise dadurch gewährleistet werden, daß in der wäßrigen Phase ein Gelgerüst aufgebaut wird, in welchem die Lipidtröpfchen stabil suspendiert sind.

Feste Stifte gemäß der Erfindung können z.B. natürliche oder synthetische Wachse, Fettalkohole oder Fettsäureester enthalten. Bevorzugt werden Lippenpflegestifte.

Bevorzugt können die erfindungsgemäßen Zubereitungen außerdem Substanzen enthalten, die UV-Strahlung im UVB-Bereich absorbieren, wobei die Gesamtmenge der Filtersubstanzen z.B. 0,1 Gew.-% bis 30 Gew.-%, vorzugsweise 0,5 bis 10 Gew.-%, insbesondere 1 bis 6 Gew.-% beträgt, bezogen auf das Gesamtgewicht der Zubereitung, um Zubereitungen zur Verfügung zu stellen, die die Haut vor dem gesamten Bereich der ultravioletten Strahlung schützen. Sie können auch als Sonnenschutzmittel dienen.

Die UVB-Filter können öllöslich oder wasserlöslich sein. Als öllösliche Substanzen sind z.B. zu nennen:
- 3-Benzylidencampher-Derivate, vorzugsweise
   3-(4-Methyl benzyliden)campher, 3-Benzylidencampher;
- 4-Aminobenzoesäure-Derivate, vorzugsweise
   4-(Dimethylamino)- benzoesäure(2-ethylhexyl)ester,
   4-(Dimethylamino)benzoesäureamylester;
- Ester der Zimtsäure, vorzugsweise
   4-Methoxyzimtsäure(2-ethylhexyl)ester,
   4-Methoxyzimtsäureisopentylester;
- Ester der Salicylsäure, vorzugsweise
   Salicylsäure(2-ethylhexyl)ester,
   Salicylsäure(4-isopropylbenzyl)ester,
   Salicylsäurehomomenthylester;
- Derivate des Benzophenons, vorzugsweise
   2-Hydroxy-4-methoxybenzophenon,
   2-Hydroxy-4-methoxy-4'-methylbenzophenon,
   2,2'-Dihydroxy-4-methoxybenzophenon;
- Ester der Benzalmalonsäure, vorzugsweise
   4-Methoxybenzalmalonsäuredi(2-ethylhexyl)ester;
- 2,4,6-Trianilino-(p-carbo-2'-ethyl-1'-hexyloxy)-1,3,5-triazin.

Als wasserlösliche Substanzen sind z.B. zu nennen:
- Salze der 2-Phenylbenzimidazol-5-sulfonsäure wie ihr Natrium-,
   Kalium- oder ihr Triethanolammonium-Salz,
   sowie die Sulfonsäure selbst;
- Sulfonsäure-Derivate von Benzophenonen, vorzugsweise
   2-Hydroxy-4-methoxybenzophenon-5-sulfonsäure und ihre Salze;
- Sulfonsäure-Derivate des 3-Benzylidencamphers, wie
   z.B. 4-(2-Oxo-3- bornylidenmethyl)benzolsulfonsäure,
   2-Methyl-5-(2-oxo-3-bornylidenmethyl)sulfonsäure und ihre Salze.

Gegenstand der Erfindung ist auch die Kombination von erfindungsgemäßen Wirkstoffen mit einem oder mehreren UVA- und/oder UVB-Filtern bzw. erfindungsgemäße kosmetische oder dermatologische Zubereitungen, welche auch einen oder mehrere UVA- und/oder UVB-Filter enthalten.

Es kann auch von besonderem Vorteil sein, die Wirkstoffe mit UVA-Filtern zu kombinieren, die auch üblicherweise in kosmetischen und/oder dermatologischen Zubereitungen enthalten sind. Bei diesen Substanzen handelt es sich vorzugsweise um Derivate des Dibenzoylmethans, insbesondere um 1-(4'-tert.Butylphenyl)-3-(4'-methoxyphenyl)propan-1,3-dion und um 1-Phenyl-3-(4'-isopropylphenyl)propan-1,3-dion. Auch diese Kombinationen bzw. Zubereitungen, die diese Kombinationen enthalten, sind Gegenstand der Erfindung. Es können die für die UVB-Kombination angegebenen Mengen eingesetzt werden.

Es werden auch vorteilhafte Zubereitungen erhalten, wenn die erfindungsgemäßen Wirkstoffe mit UVA- und UVB-Filtern kombiniert werden.

Auch Kombinationen von den erfindungsgemäßen Wirkstoffen mit einem oder mehreren Antioxidantien und einem oder mehreren UVA-Filtern und/oder einem oder mehren UVB-Filtern sind erfindungsgemäß besonders vorteilhaft.

Die kosmetischen oder dermatologischen Zubereitungen können auch anorganische Pigmente enthalten, die üblicherweise in der Kosmetik zum Schutze der Haut vor UV-Strahlen verwendet werden. Dabei handelt es sich um Oxide des Titans, Zinks, Zirkoniums, Siliciums, Mangans, Aluminiums, Cers und Mischungen davon, sowie Abwandlungen, bei denen die Oxide die aktiven Agentien sind. Besonders bevorzugt handelt es sich um Pigmente auf der Basis von Titandioxid.

Die erfindungsgemäßen Zusammensetzungen enthalten gegebenenfalls die in der Kosmetik üblichen Zusatzstoffe, beispielsweise Parfüm, Verdicker, Farbstoffe, Desodorantien, antimikrobielle Stoffe, rückfettende Agentien, Perlglanzagentien, Pflanzenextrakte, Vitamine, Wirkstoffe und dergleichen.

Alle Mengenangaben, Anteile und Prozentanteile sind, soweit nicht anders angegeben, auf das Gewicht und die Gesamtmenge bzw. auf das Gesamtgewicht der Zubereitungen bezogen.

Die nachfolgenden Beispiele sollen die vorliegende Erfindung verdeutlichen, ohne sie einzuschränken.

Die Zubereitungen werden zunächst ohne die verkapselten Wirkstoffe in üblicher Weise hergestellt. Emulsionen werden z.B. aus getrennt hergestellter Wasserphase und Fettphase durch emulgieren erhalten. Andere Zubereitungen wie z.B. Gele erhält man durch Mischen der Bestandteile.

In diese fertigen Grundlagen werden die wirkstoffhaltigen Kapseln schonend untergemischt, vorzugsweise bei Temperaturen im Bereich von Raumtemperatur bis 45°C. Als Mischwerkzeuge sind z.B. übliche Mischer geeignet, die mit niedrigen Drehzahlen betrieben werden.

Die Mengenangaben in den Beispielen sind Gewichtsprozent.

Es werden die genannten wirkstoffhaltigen Kapseln mit den angegebenen Gewichtsmengen eingesetzt. Der Wirkstoffgehalt ist in der vorstehenden Beschreibung genannt und beträgt für Cyclosytem Complex mit Vitamin A-Palmitat (IRA) 25 Gew.-% und in den übrigen Fällen jeweils 1,5 Gew.-%, jeweils bezogen auf das Gesamtgewicht der wirkstoffhaltigen Kapseln.

| Beispiel 1 Stay-in-Kur | | | | |
|---|---|---|---|---|
| | I | II | III | IV |
| Hydroxyethylcellulose | 2,0 | 2,0 | 2,0 | 2,0 |
| Cetrimoniumchlorid | 1,5 | 1,5 | 1,5 | 1,5 |
| PEG-40-hydriertes Rizinusöl | 0,4 | 0,4 | 0,4 | 0,4 |
| Polyamide millicapsules mit Vitamin E (Lipotec) | 0,3 | | 0,2 | |
| Polyamide millicapsules mit Vitamin A (Lipotec) | | 0,4 | | 0,3 |
| EDTA | 0,5 | 0,75 | | |
| NTA | | | 0,7 | 0,9 |
| Konservierungsmittel, Parfüm | q.s. | q.s. | q.s. | q.s. |
| Wasser, VES (vollentsalzt) | ad 100,00 | ad 100,00 | ad 100,00 | ad 100,00 |

| Beispiel 2 Haarlotion | | | | |
|---|---|---|---|---|
| | I | II | III | IV |
| Polyquaternium-10 | 0,3 | 0,3 | 0,3 | 0,3 |
| Ethanol | 0,8 | 0,8 | 0,8 | 0,8 |
| PEG-40-hydriertes Rizinusöl | 0,3 | 0,3 | 0,3 | 0,3 |
| Unisphären mit Vitamin E (Induchem) | 0,3 | 0,2 | | |
| Unispähren mit Vitamin A (Induchem) | | | 0,1 | 0,2 |
| EDTA | 0,4 | | 0,25 | |
| DTPA | | 0,2 | | 0,25 |
| Konservierungsmittel, Parfüm | q.s. | q.s. | q.s. | q.s. |
| Wasser, VES | ad 100,00 | ad 100,00 | ad 100,00 | ad 100,00 |

| Beispiel 3 Haarkur | | | | |
|---|---|---|---|---|
| | I | II | III | IV |
| Hydroxypropylmethylcellulose | 0,5 | 0,5 | 0,5 | 0,5 |
| Cetrimoniumbromid | 1,0 | 1,0 | 1,0 | 1,0 |
| Glycerin | 6.0 | 6,0 | 6,0 | 6,0 |
| Cetearylalkohol | 3,5 | 3,5 | 3,5 | 3,5 |
| Glycerylstearat | 3,0 | 3,0 | 3,0 | 3,0 |
| Unisphären mit Vitamin E (Induchem) | 0,5 | 0,3 | 0,25 | |
| Cyclosystem Complex mit Vitamin A-palmitat (IRA) | | | 0,6 | 0,7 |
| EDTA | 0,6 | | 0,7 | |
| NTA | | 0,5 | | 0,85 |
| Konservierungsmittel, Parfüm | q.s. | q.s. | q.s. | q.s. |
| Wasser, VES | ad 100,00 | ad 100,00 | ad 100,00 | ad 100,00 |

| Beispiel 4 Haarspülung | | | | |
|---|---|---|---|---|
| | I | II | III | IV |
| Behentrimmoniumchlorid | 1,5 | 1,5 | 1,5 | 1,5 |
| Glycerin | 5,0 | 5,0 | 5,0 | 5,0 |
| Hydroxyethylcellulose | 0,2 | 0,2 | 0,2 | 0,2 |
| Cetearylalkohol | 4,0 | 4,0 | 4,0 | 4,0 |
| Cubosome mit Methyl-beta-Cyclosystem mit Vitamin E-nicotinat | 0,45 | | 0,25 | |
| Polyamide millicapsules mit Vitamin A (Lipotec) | | 0,6 | | 0,7 |
| EDTA | 0,6 | 0,7 | | |
| EDTA | | | 0,15 | 0,3 |
| Konservierungsmittel, Parfürm | q.s. | q.s. | q.s. | q.s. |
| Wasser, VES | ad 100,00 | ad 100,00 | ad 100,00 | ad 100,00 |

| Beispiel 5 Shampoo | | | | |
|---|---|---|---|---|
| | I | II | III | IV |
| Polyquaternium-10 | 7,0 | 7,0 | 7,0 | 7,0 |
| Natriumlaurethsulfat | 9,0 | 9,0 | 9,0 | 9,0 |
| Dinatriumlaurethsulfosuccinat | 1,5 | 1,5 | 1,5 | 1,5 |
| Perlglanzmittel | 3,0 | 3,0 | 3,0 | 3,0 |
| Cocoamidopropylbetain | 2,0 | 2,0 | 2,0 | 2,0 |
| Phytosphären mit Vitamin E-acetat (Coletica) | 0,2 | 0,25 | | |
| Phytosphären mit Vitamin A (Coletica) | | | 0,25 | 0,3 |
| EDTA | 0,3 | | | 0,45 |
| HOEDTA | | 0,3 | 0,4 | |
| Konservierungsmittel, Parfüm | q.s | q.s. | q.s. | q.s. |
| Verdicker Wasser, VES | ad 100,00 | ad 100,00 | ad 100,00 | ad 100,00 |

## Patentansprüche

1. Kosmetische und dermatologische topische Zubereitungen mit einem oxidationsempfindlichen Wirkstoff oder mehreren oxidationsempfindlichen Wirkstoffen in verkapselter Form in Kombination mit einer Verbindung oder mehreren Verbindungen aus der Gruppe der Komplexbildner.

2. Verwendung von einem oxidationsempfindlichen Wirkstoff oder mehreren oxidationsempfindlichen Wirkstoffen in verkapselter Form in Kombination mit einer Verbindung oder mehreren Verbindungen aus der Gruppe der Komplexbildner in kosmetischen und dermatologisch topischen Zubereitungen, wobei der Komplexbildner zur Stabilisierung der verkapselten oxidationsempfindlichen Wirkstoffe dient.

3. Verfahren zur Herstellung der topischen Zubereitungen gemäß Anspruch 1, dadurch gekennzeichnet, daß man in an sich bekannter Weise ohne Wirkstoffkapseln die kosmetischen oder dermatologischen topischen Zusammensetzungen herstellt, und dann erst die Wirkstoffkapseln zufügt und in einem schonenden Mischverfahren mit der Grundlage vermischt.

4. Zubereitungen und Verwendung gemäß den Ansprüchen 1 - 2, dadurch gekennzeichnet, daß die oxidationsempfindlichen Wirkstoffe Antioxidantien sind und vorzugsweise aus der Gruppe der Retinoide und/oder Tocopherole gewählt werden und vorzugsweise Vitamin E oder Retinylpalmitat gewählt wird.

5. Zubereitungen und Verwendung gemäß den Ansprüchen 1 - 2, dadurch gekennzeichnet, daß die Komplexbildner aus der Gruppe der Aminopolycarbonsäuren, insbesondere der Ethylenaminopolycarbonsäuren gewählt werden und vorzugsweise EDTA gewählt wird.
